# EUROPÄISCHE PATENTANMELDUNG

(11) **EP 4 613 312 A1**
(43) Veröffentlichungstag der Anmeldung: **10.09.2025**
(21) Anmeldenummer: 24161557.4
(22) Anmeldetag: 05.03.2024
(51) Int. Cl.: A61M 16/10

(54) **MODULARES SAUERSTOFFVERSORGUNGSSYSTEM MIT MINDESTENS ZWEI SAUERSTOFFVERSORGUNGSVORRICHTUNGEN**

(71) Anmelder: Axnar Med Tech GmbH, 22453 Hamburg (DE)
(72) Erfinder: HÖLSCHER, Jochen, 22453 Hamburg (DE)
(74) Vertreter: Bardehle Pagenberg Partnerschaft mbB Patentanwälte Rechtsanwälte

(57) **Zusammenfassung**

Gegenstand der Erfindung ist ein modulares Sauerstoffversorgungssystem zur Versorgung eines Patienten. Das Sauerstoffversorgungssystem weist mindestens zwei Sauerstoffversorgungsvorrichtungen auf, wobei eine Sauerstoffversorgungsvorrichtung jeweils mindestens einen Sauerstoffkonzentrator oder mindestens einen chemischen Sauerstoffgenerator umfasst. Jede Sauerstoffversorgungsvorrichtung hat jeweils mindestens eine Steuereinheit, die den Betrieb der Sauerstoffversorgungsvorrichtung steuert und regelt. Erfindungsgemäß erfolgt somit die Kombination von zwei oder mehr Sauerstoffversorgungsvorrichtungen, die sich nicht in einem Gerät befinden und über eine Master-Slave-Steuerung verbunden sind.

## Beschreibung

Die Erfindung betrifft ein modulares Sauerstoffversorgungssystem zur Versorgung eines Patienten gemäß dem Oberbegriff von Anspruch 1.

Zur Sauerstoffversorgung von Patienten, insbesondere bei der Notfallversorgung, wird Sauerstoff aus Flaschen oder aus einem Sauerstofferzeuger, wie z.B. einem Sauerstoffkonzentrator oder Sauerstoffgenerator, eingesetzt. Ein Sauerstoffkonzentrator saugt die Luft aus seiner unmittelbaren Umgebung über eine Pumpe an. Die angesaugte Luft wird verdichtet. Der in der Luft enthaltene Stickstoff wird durch einen Adsorber, das sogenannte Molekularsieb, adsorbiert und von der restlichen Luft abtrennt. Somit erhöht sich die Sauerstoffkonzentration in der abgegebenen Luft von > 21% auf bis zu 96%.

Um bei einem Sauerstoffkonzentrator die kontinuierliche und stetige Zufuhr von konzentriertem Sauerstoff zu gewährleisten, arbeiten Sauerstoffkonzentratoren mit zwei, der oben beschriebenen Systemen parallel, sodass sich immer ein System in der Phase Ansaugen, Konzentration und Regeneration befindet, und dadurch die stetige Zufuhr von konzentriertem Sauerstoff zum Patienten gewährleistet ist. Auch bei einer stetigen Zufuhr ist aber die kurzfristig verfügbare maximale Menge Sauerstoff durch die Kapazität der Adsorber begrenzt.

Ein chemischer Sauerstoffgenerator ist ein Gehäuse, häufig ein Metallzylinder, der Metallsalzwie z.B. Natriumchlorat enthält. Durch eine Zündvorrichtung wird das nahe der Zündwärme befindliche Salz zur Reaktion gebracht und es beginnt eine chemische Reaktion, die schnell Sauerstoff freisetzt. Die Reaktion ist stark exotherm, so dass im Gehäuse Temperaturen von über 400°C oder sogar bis zu 600°C entstehen.

Ale bekannten Sauerstoffversorgungsgeräte haben somit für sich gesehen Vorteile und Nachteile für den einzelnen Einsatzzweck. Ein Sauerstoffgerät mit einem Sauerstoffkonzentrator, der ein Druckwechseladsorptionssystem (PSA) oder ein Vakuum- Druckwechseladsorptionssystem (VPSA) hat, benötigt eine gewisse Mindestgröße, um eine ausreichende Menge Sauerstoff zu erzeugen. Es ist daher schwierig ein Gerät so klein und leicht zu konstruieren, dass es transportabel ist und leicht zum Einsatzort transportiert werden kann. Bei einem chemischen Sauerstoffgenerator ist hingegen die abgegebene Menge Sauerstoff nicht regulierbar, da mit Start der chemischen Reaktion immer die Maximalmenge abgegeben wird. Zudem erhitzt sich der Sauerstoffgenerator bei der Reaktion auf bis zu 400°C oder sogar 600°C, so dass eine Handhabung in transportablen Einheiten schwierig ist

Aus DE 102 05 955 A1 ist eine Vorrichtung zur Bereitstellung von Atemgas bekannt, die den von einem Sauerstoffgenerator erzeugten Sauerstoff einer Druckgasflasche zuführt. Im Sauerstoffgenerator erfolgt eine elektrolytische Zersetzung von Wasser in Wasserstoff und Sauerstoff. Der erzeugte Sauerstoff wird komprimiert und in die Druckgasflasche eingespeist. Hierdurch soll auch bei Einsatz eines Sauerstoffgenerators eine gleichmäßige Sauerstoffzufuhr ermöglicht werden.

Aufgabe der vorliegenden Erfindung ist es daher, eine Sauerstoffversorgungsvorrichtung bereitzustellen, die die vorstehenden Nachteile überwindet und die insbesondere konstant eine regulierbare Menge Sauerstoff zur Verfügung stellt.

Hauptmerkmale der Erfindung sind im kennzeichnenden Teil von Anspruch 1 angegeben. Ausgestaltungen sind Gegenstand der Ansprüche 2 bis 12.

Weitere Ausführungsformen sind Gegenstand der Unteransprüche oder nachfolgend beschrieben.

Das erfindungsgemäße modulare Sauerstoffversorgungssystem zur Versorgung eines Patienten weist mindestens zwei Sauerstoffversorgungsvorrichtungen auf, wobei eine Sauerstoffversorgungsvorrichtung jeweils mindestens einen Sauerstoffkonzentrator oder mindestens einen chemischen Sauerstoffgenerator oder mindestens einen elektrochemischen Sauerstoffgenerator umfasst. Jede Sauerstoffversorgungsvorrichtung weist jeweils mindestens eine Steuereinheit auf, die den Betrieb der Sauerstoffversorgungsvorrichtung steuert und ggf. regelt. Die Steuereinheiten der Sauerstoffversorgungsvorrichtungen sind alle untereinander verbunden, so dass Kommunikationssignale mittels Kabel oder kabellos zwischen den Steuereinheiten übertragen werden. Eine erste Sauerstoffversorgungsvorrichtung ist als Hauptgerät (Master) geschaltet und eine zweite und jede weitere Sauerstoffversorgungsvorrichtung ist als untergeordnetes Gerät (Slave) geschaltet. Das Hauptgerät steuert die untergeordneten Geräte. Nur das Hauptgerät ist direkt oder indirekt mit einem Patienten, indirekt z.B. über eine Atemhilfe, verbunden, während die untergeordneten Geräte mit dem Hauptgerät verbunden sind.

In einem Master-Slave-System wird die Kommunikation zwischen mehreren Geräten und der Zugriff der Geräte auf Ressourcen geregelt. Das Hauptgerät (Master) übernimmt die Steuerung eines oder mehrerer Slaves und erteilt die Zugriffs- oder Kommunikationsberechtigungen. Die Initiative geht immer vom Hauptgerät aus und die Kontrollrichtung immer vom Master in Richtung der Slaves. Slaves können nicht selbständig eine Kommunikation anstoßen oder den Zugriff auf Ressourcen initiieren.

Erfindungsgemäß erfolgt somit die Kombination von zwei oder mehr Sauerstoffversorgungsvorrichtungen, die sich nicht in einem Gerät befinden und über eine Master-Slave-Steuerung verbunden sind. Bevorzugt weist das modulare Sauerstoffversorgungssystem zwei bis 5, besonders bevorzugt zwei bis drei miteinander verbundene Sauerstoffversorgungseinrichtungen auf.

In einer Ausführungsform umfasst die mindestens eine Sauerstoffversorgungsvorrichtung:
- mindestens einen Sauerstoffkonzentrator,
- mindestens einen Lufteinlass, durch den Umgebungsluft in die Sauerstoffversorgungsvorrichtung transportiert wird,
- mindestens einen Sauerstoff-Auslass, durch den angereicherter Sauerstoff aus der Sauerstoffversorgungsvorrichtung transportiert wird,
- mindestens einen Abluftauslass, durch den Abluft aus der Sauerstoffversorgungsvorrichtung transportiert wird,
- ein Gasleitungssystem, das mit dem mindestens einen Lufteinlass, dem Auslass und dem Sauerstoffkonzentrator fluidisch verbunden ist,
- eine Stromquelle,
- mindestens eine Steuereinheit,
- mindestens eine Pumpe und
- ein Gehäuse, wobei alle Komponenten der Sauerstoffversorgungsvorrichtung im oder am Gehäuse angeordnet sind.
Der Lufteinlass weist einen Ansaugstutzen auf, durch den die Umgebungsluft in die Sauerstoffversorgungsvorrichtung gesogen wird.

In dieser Ausführungsform ist der Sauerstoffkonzentrator bevorzugt ein Druckwechseladsorptionssystem (PSA) oder ein Vakuum- Druckwechseladsorptionssystem (VPSA) und weist eine oder mehrere Adsorbersäulen auf.

Mindestens eine Sauerstoffversorgungsvorrichtung, bevorzugt die zweite oder dritte Sauerstoffversorgungsvorrichtung kann ein chemischer Sauerstoffgenerator sein. Der chemische Sauerstoffgenerator weist auf:
- eine Sauerstofferzeugungseinheit zum chemischen Erzeugen von Sauerstoff,
- eine Zündeinrichtung zum Zünden der Sauerstofferzeugungseinheit, um die Sauerstofferzeugung zu starten,
- ein Gehäuse, wobei alle Komponenten der Sauerstoffversorgungsvorrichtung im oder am Gehäuse angeordnet sind, und
- ein Gasleitungssystem, das mit einem Auslass und dem Sauerstoffgenerator fluidisch verbunden ist.

Die Sauerstofferzeugungseinheit ist bevorzugt austauschbar, da es sich um eine einmal verwendbare Einheit handelt, die nach erfolgter chemischer Reaktion entsorgt werden muss. Sie kann daher als Austauscheinheit, d.h. Nachfülleinheit ausgebildet sein, so dass der Sauerstoffgenerator erneut verwendet werden kann. Die Steuereinheit des chemischen Sauerstoffgenerators kann ein Signal an einen Auslöseeinheit mit einem Auslösemechanismus, z.B. federgesteuert, geben, der das Zünden der Zündeinrichtung auslöst.

Bevorzugt ist der chemische Sauerstoffgenerator als untergeordnetes Gerät geschaltet. Er kann somit bei Bedarf kurzfristig eine hohe Menge Sauerstoff zur Verfügung stellen, wenn das Hauptgerät einen Bedarf meldet und die Sauerstofferzeugung initiiert.

Die chemische Reaktion im chemischen Sauerstoffgenerator führt dazu, dass die Sauerstoffversorgungsvorrichtung nach der Zündung für mehrere Minuten Sauerstoff produziert, bevor die reaktiven Bestandteile in der Sauerstofferzeugungseinheit verbraucht sind. Bevorzugt enthält die Sauerstofferzeugungseinheit als sauerstofferzeugenden chemischen Stoff ein oder mehrere Metallchlorate oder Metallperchlorate. Der Sauerstoffgenerator kann auch stufenweise ausgebildet sein, d.h. er weist mehrere räumlich von der Zündeinrichtung weg in Stufen angeordnete Brennstufen auf.

Die Sauerstoffversorgungsvorrichtung kann mindestens ein Reservoir zur Aufnahme von konzentriertem Sauerstoff aufweisen. Die Sauerstoffversorgungsvorrichtung weist bevorzugt ein Reservoir auf, wenn sie als Master geschaltet ist, besonders bevorzugt wenn das untergeordnete Gerät ein chemischer Sauerstoffgenerator ist.

Die Sauerstoffversorgungsvorrichtungen können einen oder mehrere Sensoren im Gehäuse aufweisen, die bevorzugt mit dem Gasleitungssystem verbunden sind.

In einer Ausführungsform weist die erste Sauerststoffversorgungsvorrichtung, die als Hauptgerät geschaltet ist, einen Sauerstoffkonzentrator auf, der ein Druckwechseladsorptionssystem (PSA) oder ein Vakuum- Druckwechseladsorptionssystem (VPSA) ist und eine zweite Sauerstoffversorgungsvorrichtung, die als untergeordnetes Gerät geschaltet ist, ist ein chemischer Sauerstoffgenerator.

Mindestens eine Sauerstoffversorgungsvorrichtung kann ein mobiles, bevorzugt tragbares, Gerät sein, das ein stoßfestes Gehäuse hat und sofern die Sauerstoffversorgungsvorrichtung einen Sauerstoffkonzentrator umfasst, als Stromquelle mindestens eine Batterieeinheit oder mindestens einen Akkumulator aufweist.

Das Hauptgerät kann eine stationäre Sauerstoffversorgungsvorrichtung sein, die in einem Rettungsmittel z.B. einem Rettungswagen, Rettungshubschrauber, Rettungsflugzeug, Einsatzfahrzeug oder Rettungsschiff, montiert ist, und das untergeordnete Gerät kann ein mobiles, bevorzugt tragbares, Gerät sein. Grundsätzlich ist ein Rettungsmittel ein Landfahrzeug, Wasserfahrzeug oder Luftfahrzeug des Rettungsdienstes.

In dieser Ausführungsform ist die mobile Sauerstoffversorgungsvorrichtung bevorzugt kleiner als die stationäre Sauerstoffversorgungsvorrichtung, d.h. die stationäre Sauerstoffversorgungsvorrichtung produziert eine größerer Menge Sauerstoff mit höherer Flussrate, z.B. 2 - 8 I, während die kleinere mobile Sauerstoffversorgungsvorrichtung eine geringere Sauerstoffmenge, z.B. von 1-5 I, produziert. Die mobile Sauerstoffversorgungsvorrichtung hat den Vorteil, dass sie an die Einsatzstelle transportiert werden kann und dort für die Oxygenierung des Patienten eingesetzt werden kann, z.B. zur Inhalation oder zur manuellen oder maschinellen Beatmung. Sobald der Patient initial versorgt ist, kann er zum Rettungsmittel transportiert werden und dort an das stationäre Hauptgerät angeschlossen werden, dass für zusätzlichen Sauerstoff mit der mobilen Sauerstoffversorgungsvorrichtung gekoppelt wird.

In einer Ausführungsform sind das Hauptgerät und mindestens ein untergeordnetes Gerät baugleich und unterscheiden sich nur durch die Schaltung als Hauptgerät (Master) oder untergeordnetes Gerät (Slave) voreinander. Nur durch die Schaltung wird eine der beiden Sauerstoffversorgungsvorrichtungen zum Hauptgerät (Master), baulich könnten aber beide als Hauptgerät fungieren und entsprechend geschaltet werden. So können bei einem Einsatz anders als bisher zwei oder mehr Sauerstoffversorgungsgeräte gekoppelt werden, um eine hohe Leistung zur Patientenversorgung bereitstellen zu können.

Das modulare Sauerstoffversorgungssystem kann genau zwei Sauerstoffversorgungsvorrichtungen aufweisen, von denen bevorzugt mindestens eine Vorrichtung einen Sauerstoffkonzentrator aufweist.

Die Sauerstoffversorgungsvorrichtung kann zusätzlich mit einem Sauerstoff-Pufferspeicher ausgestattet sein, in dem überschüssiger Sauerstoff gespeichert wird.

Das erfindungsgemäße modulare Sauerstoffversorgungssystem ermöglicht eine bessere bedarfsgerechte Sauerstoffversorgung im Vergleich mit Einzelgeräten. Wenn beispielsweise zwei Sauerstoffversorgungsvorrichtung gekoppelt sind, die als Hauptgerät eine Sauerstoffversorgungseinrichtung mit einem Vakuum- Druckwechseladsorptionssystem umfassen, und dieses Hauptgerät mit einem chemischen Sauerstoffgenerator als untergeordnetem Gerät verbunden ist, so kann das untergeordnete Gerät als Booster kurzfristig eine höhere Sauerstoffmenge bereitstellen. Bisher war eine Kopplung von zwei Sauerstoffversorgungsgeräten nur über ein Y-Stück bekannt. Dieses entspricht einer Kopplung von zwei untergeordneten Geräten (Slave - Slave - Verbindung). Der Einsatz einer solchen Koppelung ist nicht zulässig, da die Sauerstoffmengen, die von beiden Geräten bereitgestellt werden, nicht aufeinander abgestimmt sind und die Versorgungsleistung nicht geregelt werden kann. Es wäre daher patientengefährdend, die zwei nicht aufeinander abgestimmten Geräte mit einem Y-Stück einzusetzen.

Bisher bestand im Stand der Technik das Problem, ein Sauerstoffversorgungsgerät mit einem PSA- oder VPSA-Sauerstoffgerät klein und transportabel zu machen und trotzdem für die mobilen Einsätze eine ausreichende Sauerstoffmenge bereit zu stellen.

Das erfindungsgemäße modulare Sauerstoffversorgungsystem erlaubt es eine Sauerstoffversorgungseinrichtung bereit zu stellen, die gleichzeitig klein und leicht ist und dadurch tragbar, z.B. ein mobiler Slave, und zusätzlich bei Akut-Bedarf eine hohe Leistung abzudecken, was bisher Widersprüche sind. Der Akut-Bedarf kann durch Koppelung mit einer weiteren Sauerstoffversorgungsvorrichtung gedeckt werden, z.B. mit einem chemischen Sauerstoffgenerator oder durch Koppelung zweier eine Sauerstoffversorgungseinrichtung auch am Einsatzort.

Das erfindungsgemäße modulare Sauerstoffversorgungsystem ermöglicht es konzentrierten Sauerstoff aus einer zweiten Sauerstoffquelle aufzunehmen und den konzentrierten Sauerstoff aus den verschiedenen Quellen zu mischen und zur Verfügung zu stellen. Hierdurch lässt sich die bereitgestellte Sauerstoffmenge besser an das jeweilige Bedürfnis anpassen.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine schematische Zeichnung eines erfindungsgemäßen Sauerstoffversorgungssystems und
- Fig. 2: eine schematische Zeichnung eines weiteren erfindungsgemäßen Sauerstoffversorgungssystems.

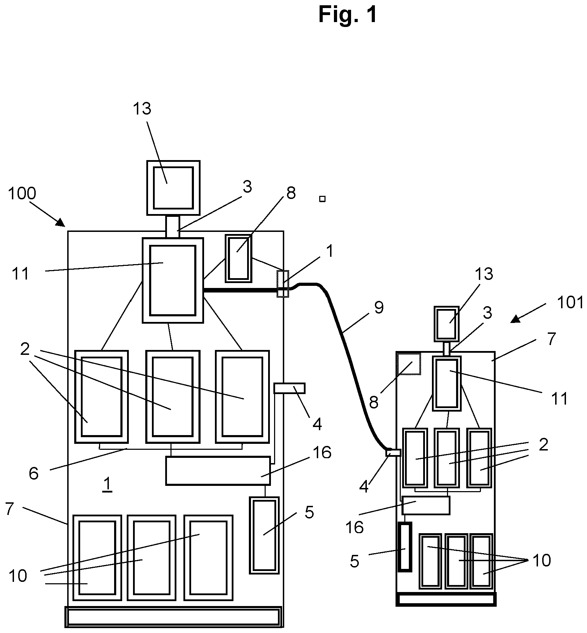

Figur 1 zeigt einen schematischen Aufbau eines erfindungsgemäßen Sauerstoffversorgungssystems. Das Sauerstoffversorgungssystem weist zwei Sauerstoffversorgungsvorrichtungen 100 und 101 auf, die mit Sauerstoffkonzentratoren ausgestattet sind. Die größere Sauerstoffversorgungsvorrichtung 100 ist als Hauptgerät geschaltet, während die kleinere Sauerstoffversorgungsvorrichtung als untergeordnetes Gerät geschaltet ist. Beide Sauerstoffversorgungsvorrichtungen haben den folgenden Aufbau: In einem Gehäuse 7 sind ein Sauerstoffkonzentrator 2, eine Steuereinheit 8, eine Stromquelle 10 und zwei Pumpen 11 und 16 angeordnet. Am Gehäuse befindet sich ein Lufteinlass 3, der durch die Gehäusewand hindurchführt. Der Lufteinlass weist einen Ansaugstutzen 13 an der Außenseite des Gehäuses auf. Der Abluftauslass 5 führt ebenfalls durch das Gehäuse 7 und ermöglicht es Abluft aus der Sauerstoffversorgungsvorrichtung zu transportieren. Der Sauerstoffauslass 4 kann beim Mastergerät 100 mit einem Schlauch verbunden werden, der zum Patienten führt. Der Sauerstoffauslass 4 des untergeordneten Geräts 101 (Slave) ist mit einer sauerstoffführenden Leitung 9 verbunden. Über diese wird Sauerstoff vom untergeordneten Gerät 101 durch einen Sauerstoffeinlass 12 für eine externe Quelle in das übergeordnete Gerät 100 (Master) geführt und kann von dort ebenfalls an den Patienten abgegeben werden.

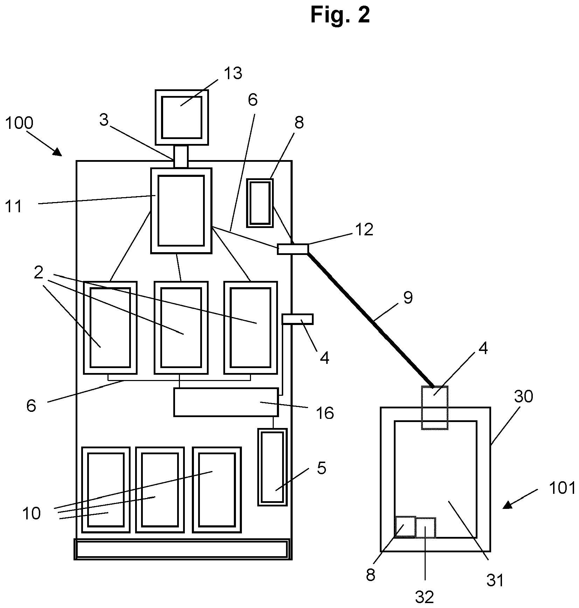

Figur 2 zeigt einen schematischen Aufbau einer weiteren Ausführungsform des erfindungsgemäßen Sauerstoffversorgungssystems. Das Sauerstoffversorgungssystem weist zwei Sauerstoffversorgungsvorrichtungen 100 und 101 auf, von denen das Hauptgerät 100 einen Sauerstoffkonzentrator umfasst und das untergeordnete Gerät 101 einen chemischen Sauerstoffgenerator 30 hat. Der chemische Sauerstoffgenerator 30 weist eine Sauerstofferzeugungseinheit 31 zum chemischen Erzeugen von Sauerstoff und eine Zündeinrichtung 32 auf. Die Zündeinrichtung 32 ist mit einer Steuereinheit 8 verbunden. Das Hauptgerät 100 und der chemische Sauerstoffgenerator 30 sind über eine Gasleitung fluidisch verbunden, durch die vom Sauerstoffgenerator erzeugter Sauerstoff an das Hauptgerät abgegeben wird.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

| | |
|---|---|
| Sauerstoffversorgungsvorrichtung | 1 |
| Sauerstoffkonzentrator | 2 |
| Lufteinlass | 3 |
| Sauerstoff-Auslass | 4 |
| Abluftauslass | 5 |
| Gasleitungssystem | 6 |
| Gehäuse | 7 |
| Ansaugstutzen | 13 |
| Steuereinheit | 8 |
| Sauerstoffführende Leitung | 9 |
| Stromquelle | 10 |
| Erste Pumpe | 11 |
| Sauerstoffeinlass für externe Quelle | 12 |
| Zweite Pumpe | 16 |
| Vakuumpumpe | 17 |
| Chemischer Sauerstoffgenerator | 30 |
| Sauerstofferzeugungseinheit | 31 |
| Zündeinrichtung | 32 |
| Erste Sauerstoffversorgungsvorrichtung | 100 |
| Zweite Sauerstoffversorgungsvorrichtung | 101 |

## Patentansprüche

1. Modulares Sauerstoffversorgungssystem zur Versorgung eines Patienten aufweisend mindestens zwei Sauerstoffversorgungsvorrichtungen,
wobei eine Sauerstoffversorgungsvorrichtung (1) jeweils mindestens einen Sauerstoffkonzentrator (2) oder mindestens einen chemischen Sauerstoffgenerator (30) oder mindestens einen elektrochemischen Sauerstoffgenerator umfasst, und
jede Sauerstoffversorgungsvorrichtung (1) jeweils mindestens eine Steuereinheit (8) aufweist, die den Betrieb der Sauerstoffversorgungsvorrichtung (1) steuert und ggf. regelt, **dadurch gekennzeichnet, dass**
die Steuereinheiten (8) der Sauerstoffversorgungsvorrichtungen (1) untereinander verbunden sind, so dass Kommunikationssignale mittels Kabel oder kabellos zwischen den Steuereinheiten (8) übertragen werden,
eine erste Sauerstoffversorgungsvorrichtung als Hauptgerät (Master) geschaltet ist und eine zweite und jede weitere Sauerstoffversorgungsvorrichtung als untergeordnetes Gerät (Slave) geschaltet ist und das Hauptgerät die untergeordneten Geräte steuert und nur das Hauptgerät mit einem Patienten oder einem Beatmungsgerät verbunden ist.

2. Modulares Sauerstoffversorgungssystem gemäß Anspruch 1, **dadurch gekennzeichnet, dass** die mindestens eine Sauerstoffversorgungsvorrichtung umfasst:
- mindestens einen Sauerstoffkonzentrator (2),
- mindestens einen Lufteinlass (3), durch den Umgebungsluft in die Sauerstoffversorgungsvorrichtung transportiert wird,
- mindestens einen Sauerstoff-Auslass (4), durch den angereicherter Sauerstoff aus der Sauerstoffversorgungsvorrichtung (1) transportiert wird,
- mindestens einen Abluftauslass (5), durch den Abluft aus der Sauerstoffversorgungsvorrichtung (1) transportiert wird,
- ein Gasleitungssystem (6), das mit dem mindestens einen Lufteinlass (3), dem Auslass (4) und dem Sauerstoffkonzentrator (2) fluidisch verbunden ist,
- eine Stromquelle (10),
- mindestens eine Steuereinheit (8),
- mindestens eine Pumpe (11, 16) und
- ein Gehäuse (7), wobei alle Komponenten der Sauerstoffversorgungsvorrichtung (1) im oder am Gehäuse (7) angeordnet sind,
wobei der Lufteinlass (3) einen Ansaugstutzen aufweist, durch den die Umgebungsluft in die Sauerstoffversorgungsvorrichtung (1) gesogen wird.

3. Modulares Sauerstoffversorgungssystem gemäß Anspruch 2, **dadurch gekennzeichnet, dass** der Sauerstoffkonzentrator (2) ein Druckwechseladsorptionssystem (PSA) oder ein Vakuum- Druckwechseladsorptionssystem (VPSA) ist und eine oder mehrere Adsorbersäulen aufweist.

4. Modulares Sauerstoffversorgungssystem gemäß Anspruch 1, **dadurch gekennzeichnet, dass** mindestens eine Sauerstoffversorgungsvorrichtung, bevorzugt die zweite oder dritte Sauerstoffversorgungsvorrichtung ein chemischer Sauerstoffgenerator (30) ist und der Sauerstoffgenerator aufweist:
- eine Sauerstofferzeugungseinheit (31) zum chemischen Erzeugen von Sauerstoff,
- eine Zündeinrichtung (32) zum Zünden der Sauerstofferzeugungseinheit, um die Sauerstofferzeugung zu starten,
- ein Gehäuse (7), wobei alle Komponenten der Sauerstoffversorgungsvorrichtung (1) im oder am Gehäuse (7) angeordnet sind, und
- ein Gasleitungssystem (6), das mit einem Auslass (4) und dem Sauerstoffgenerator (2) fluidisch verbunden ist,

5. Modulares Sauerstoffversorgungssystem gemäß Anspruch 4, **dadurch gekennzeichnet, dass** der chemische Sauerstoffgenerator (30) als untergeordnetes Gerät geschaltet ist.

6. Modulares Sauerstoffversorgungssystem gemäß einem der vorhergehenden Ansprüche, **dadurch gekennzeichnet, dass** die Sauerstoffversorgungsvorrichtungen einen oder mehrere Sensoren im Gehäuse aufweist, die bevorzugt mit dem Gasleitungssystem verbunden sind.

7. Modulares Sauerstoffversorgungssystem gemäß einem der vorhergehenden Ansprüche, **dadurch gekennzeichnet, dass** die erste Sauerststoffversorgungsvorrichtung, die als Hauptgerät geschaltet ist, einen Sauerstoffkonzentrator (2) aufweist, der ein Druckwechseladsorptionssystem (PSA) oder ein Vakuum- Druckwechseladsorptionssystem (VPSA) ist und eine zweite Sauerstoffversorgungsvorrichtung, die als untergeordnetes Gerät geschaltet ist, ein chemischer Sauerstoffgenerator (30) ist.

8. Modulares Sauerstoffversorgungssystem gemäß einem der vorhergehenden Ansprüche, **dadurch gekennzeichnet, dass** mindestens eine Sauerstoffversorgungsvorrichtung (1) ein mobiles Gerät ist, das ein stoßfestes Gehäuse (7) hat und ggf. als Stromquelle mindestens eine Batterieeinheit (10) oder einen Akkumulator (10) aufweist.

9. Modulares Sauerstoffversorgungssystem gemäß einem der vorhergehenden Ansprüche, **dadurch gekennzeichnet, dass** das Hauptgerät eine stationäre Sauerstoffversorgungsvorrichtung ist, die in einem Rettungsmittel montiert ist, und das untergeordnete Gerät ein tragbares, mobiles Gerät ist.

10. Modulares Sauerstoffversorgungssystem gemäß einem der Ansprüche 1 bis 8, **dadurch gekennzeichnet, dass** das Hauptgerät und mindestens ein untergeordnetes Gerät baugleich sind.

11. Modulares Sauerstoffversorgungssystem gemäß einem der vorhergehenden Ansprüche, **dadurch gekennzeichnet, dass** das modulare Sauerstoffversorgungssystem genau zwei Sauerstoffversorgungsvorrichtungen aufweist.

12. Modulares Sauerstoffversorgungssystem gemäß einem der vorhergehenden Ansprüche, **dadurch gekennzeichnet, dass** mindestens eine Sauerstoffversorgungsvorrichtung mindestens ein Reservoir zur Aufnahme von konzentriertem Sauerstoff aufweist.
